# FASCICULE DE BREVET EUROPEEN

(11) **EP 2 603 163 B1**
(45) Date de publication et mention de la délivrance du brevet: **12.04.2017**
(21) Numéro de dépôt: 11767239.4
(22) Date de dépôt: 05.10.2011
(51) Int. Cl.: A61C 8/00

(54) **IMPLANT ENDO-OSSEUX À ANCRAGE AMÉLIORÉ**
ENOSSALES IMPLANTAT MIT VERBESSERTER VERANKERUNG
ENDOSSEOUS IMPLANT HAVING IMPROVED ANCHORAGE

(43) Date de publication de la demande: 19.06.2013
(73) Titulaire: LACAZE, Guillaume, 1007 Lausanne (CH)
(72) Inventeur: LACAZE, Guillaume, 82700 Montech (FR)
(74) Mandataire: Ipside
(86) Numéro de dépôt international: PCT/EP2011/067410
(87) Numéro de publication internationale: WO 2012/045787

(56) Documents cités:
- DE-U1- 29 619 163
- FR-A1- 2 467 584
- KR-A- 20040 063 528
- US-A- 5 087 199
- US-A1- 2005 042 574
- US-A1- 2010 114 314

## Description

L'invention relève du domaine des implants endo-osseux destinés à des applications dentaires, orthopédiques, chirurgicales ou ostéoplastiques, tels que des implants dentaires, de hanches, de genoux ou articulaires.

Les implants endo-osseux sont utilisés en chirurgie pour implanter un dispositif prothétique chez un patient, tel que par exemple une prothèse dans le domaine dentaire. De tels implants endo-osseux sont principalement utilisés dans le domaine dentaire notamment, mais aussi dans le domaine de la chirurgie réparatrice par exemple. Ces domaines d'application sont donnés à titre d'exemple et ne sont pas restrictifs quant à la portée de la présente invention.

Un implant endo-osseux est généralement constitué d'un corps de forme allongée destiné à être implanté dans un logement formé dans un tissu osseux, tel que l'os de la mâchoire pour une application dentaire par exemple, et qui comporte, d'une part, des moyens d'ancrage à l'intérieur du tissu osseux et d'autre part, des moyens pour recevoir un dispositif prothétique qui peut être un faux moignon, un pilier gingival, une bague,....

Ce type d'implant endo-osseux doit pouvoir être facilement introduit dans le tissu osseux et se lier fortement à celui-ci.

Il doit également être facilement réalisable et présenté un faible coût de fabrication.

Le document US 2010/0114314 A1 décrit par ailleurs un implant dentaire avec un partie d'accrochage et une partie d'expansion. A l'heure actuelle, la pose d'un ensemble prothétique complet (constitué d'un implant endo-osseux et d'un dispositif prothétique associé) est relativement longue. Dans un premier temps, l'implant endo-osseux est mis en place dans le logement formé dans le tissu osseux. Cette mise en place de l'implant endo-osseux dans le tissu osseux est toujours suivie d'une période d'ostéointégration comprise généralement entre 5 et 6 mois afin de permettre à l'os de venir adhérer à l'implant endo-osseux. Puis, dans un deuxième temps, en fonction de l'application et dudit implant endo-osseux, celui-ci devient porteur d'un dispositif prothétique spécifique.

Cette période d'ostéointégration avant de pouvoir fixer le dispositif prothétique sur l'implant endo-osseux est généralement mal vécue par le patient à qui l'on vient de poser l'implant endo-osseux, principalement dans le cas d'un implant endo-osseux dentaire. En effet, pendant cette période, comme il n'est pas possible de placer de dispositif prothétique sur l'implant endo-osseux, cet emplacement reste vide et la dentition souffre d'un aspect inesthétique, surtout lorsque cet emplacement est situé au niveau des incisives.

Un autre inconvénient de l'implant endo-osseux actuel est la liaison entre l'implant endo-osseux et le dispositif prothétique qui présente un niveau d'étanchéité insuffisant. Ceci peut conduire à des problèmes de contamination bactériologique, à des infiltrations d'impuretés à l'intérieur de l'implant endo-osseux et provoquer des inflammations de la gencive, éventuellement une détérioration de l'os.

Un autre inconvénient de l'implant endo-osseux actuel est le problème de la qualité de son ancrage dans le tissu osseux.

En effet, l'ancrage d'un implant endo-osseux, lors de sa mise en place par le praticien à l'intérieur du logement préalablement ménagé dans le tissu osseux, est actuellement uniquement réalisé à partir de moyens d'ancrage agencés sur une surface extérieure du corps.

Les moyens d'ancrage sont par exemple du type par impaction ou préférentiellement du type par vissage, généralement auto-taraudant. D'une manière générale, ces moyens d'ancrage mettent en oeuvre des reliefs de fixation qui sont agencés pour maintenir l'implant endo-osseux en position à l'intérieur de l'os lors de sa mise en place. De tels reliefs de fixation sont par exemple constitués d'un filetage, pour les moyens d'ancrage par vissage, et/ou d'organes annulaires, pour les moyens d'ancrage par impaction.

Or, dans le domaine dentaire, il est fréquent que l'implant endo-osseux soit soumis à des pressions et/ou de fortes contraintes, et/ou des surcharges. Ainsi, par exemple, l'implant endo-osseux peut subir des rotations inopportunes, accidentelles suite à une mastication, ou encore engendrées par la langue du patient aptes à provoquer un retrait inopiné de l'implant endo-osseux hors du logement du tissu osseux.

Ces moyens d'ancrage ne sont donc pas suffisants pour éviter tout risque de retrait spontané de l'implant endo-osseux pendant la période d'ostéointégration.

Il existe donc un réel besoin d'améliorer l'étanchéité et l'ancrage des implants endo-osseux actuels, tout en conservant une réalisation aisée de l'implant et à faible coût.

La présente invention a pour but de pallier aux inconvénients précédemment évoqués et consiste pour cela en un implant endo-osseux, tel que défini dans la revendication 1.

Les moyens de liaison mécanique sont aptes à rendre les parties d'accrochage et d'expansion solidaires entre elles.

Ainsi, l'implant endo-osseux présente, à l'état initial, c'est-à-dire avant son insertion dans un logement de réception crée dans le tissu osseux et adapté aux dimensions de l'implant endo-osseux, un dispositif de fixation avec la partie d'accrochage ne présentant aucun élargissement. Lors de l'introduction de l'implant endo-osseux dans le logement de réception, le dispositif de fixation n'est pas en contact avec des parois dudit logement de réception.

Lorsque la partie d'expansion est en contact avec les parois du logement, un déplacement relatif entre lesdites deux parties est créé provoquant un élargissement de la partie d'accrochage jusqu'à ce que ladite partie d'accrochage s'ancre dans les parois du logement du tissu osseux.

La présence d'un tel dispositif de fixation sur l'implant endo-osseux permet ainsi d'améliorer l'ancrage dudit implant endo-osseux dans le tissu osseux par une immobilisation tant en rotation qu'en translation dans ledit tissu osseux. Ainsi, l'implant endo-osseux selon l'invention ne subit plus de rotations ou de translations accidentelles ou inopportunes aptes à provoquer le retrait involontaire de l'implant endo-osseux hors du logement du tissu osseux contrairement aux implants endo-osseux de l'art antérieur.

L'implant endo-osseux tel que proposé trouve préférentiellement application dans le domaine dentaire pour l'installation de prothèses de dents mais l'implant endo-osseux trouve également application dans de nombreux domaines, tels que à titre d'exemples non restrictifs, les domaines orthopédiques, chirurgicales, ostéoplastiques pour l'installation de prothèses, de hanches, de genoux ou articulaires.

Suivant des modes de réalisation préférés, l'invention répond en outre aux caractéristiques suivantes, mises en oeuvre séparément ou en chacune de leurs combinaisons techniquement opérantes avec l'objet de l'invention tel que défini dans les revendications.

L'implant endo-osseux comprend un corps présentant une forme allongée selon un axe longitudinal. Ledit corps est préférentiellement, compris entre une première extrémité, dite extrémité exo-osseuse et une deuxième extrémité, dite extrémité endo-osseuse.

Le dispositif de fixation est préférentiellement lié à une des extrémités dudit corps, préférentiellement à l'extrémité endo-osseuse.

Dans un mode de réalisation de l'implant endo-osseux, ledit implant endo-osseux comporte, au niveau d'une surface externe du corps, des moyens d'ancrage au tissu osseux.

Dans un exemple préféré de réalisation des moyens d'ancrage, lesdits moyens d'ancrage sont du type par vissage et comportent un filetage sur la surface externe.

Dans un mode de réalisation de l'implant endo-osseux, ledit implant endo-osseux comporte, au niveau de l'extrémité exo-osseuse, des premiers moyens de connexion aptes à coopérer avec une clé de vissage pour l'insertion de l'implant endo-osseux dans le logement et des deuxièmes moyens de connexion aptes à coopérer avec un dispositif prothétique.

Dans des exemples de réalisation des deuxièmes moyens de connexion, lesdits moyens peuvent être de forme polygonale, filetés interne ou externe ou toute autre forme permettant la rétention d'un dispositif prothétique.

Selon une caractéristique de l'invention, le au moins un degré de liberté entre la partie d'accrochage et la partie d'expansion est une translation selon l'axe longitudinal. Les parties d'accrochage et d'expansion sont ainsi mobiles l'une par rapport à l'autre, en mouvement de translation suivant l'axe longitudinal et le mouvement de translation permet d'élargir la partie d'accrochage transversalement par rapport audit axe longitudinal jusqu'à ce que ladite partie d'accrochage accroche aux parois du logement du tissu osseux.

Selon une autre caractéristique de l'invention, la partie d'accrochage est déformable élastiquement et comporte au moins deux fentes longitudinales aptes à permettre un élargissement plus important de ladite partie d'accrochage.

Selon une autre caractéristique de l'invention, la partie d'accrochage comporte, au niveau d'une surface externe, des moyens d'immobilisation en rotation et/ou en translation dudit l'implant endo-osseux dans le tissu osseux après l'implantation.

Dans un exemple de réalisation des moyens d'immobilisation, lesdits moyens d'immobilisation sont un filetage, des aspérités, des dents ou des saillies, à profil préférentiellement triangulaire.

Selon l'implant endo-osseux de l'invention, la partie d'expansion est fixée au corps et la partie d'accrochage est agencée circonférentiellement autour de ladite partie d'expansion et les moyens de liaison sont des moyens de vissage coopérants et le dispositif de fixation comporte des moyens anti-retour coopérants aptes à empêcher la rotation entre la partie d'accrochage et la partie d'expansion en sens inverse à celui imparti par lesdits moyens de vissage.

Les moyens de vissage permettent un vissage, suivant un sens de rotation donné, d'une partie par rapport à l'autre partie du dispositif de fixation. Les moyens anti-retour empêchent la rotation entre les deux parties en sens inverse.

Selon une caractéristique avantageuse de l'invention, les moyens anti-retour coopérants comportent, d'une part, au niveau d'une surface externe de la partie d'expansion, une première denture périphérique extérieure et, d'autre part, au niveau d'une surface interne de la partie d'accrochage, une deuxième denture périphérique extérieure.

Dans un exemple de réalisation des dentures, lesdites dentures périphériques extérieures de la partie d'accrochage et de la partie d'expansion, ont un profil transversal en dents de scie à flancs asymétriques, le profil comportant des flancs à pente montante et des flancs à pente descendante, chaque flanc à pente montante étant relié à un flanc à pente descendante par une arête sensiblement tranchante.

Les dentures périphériques extérieures sont donc disposées l'une par rapport à l'autre de sorte à ne pouvoir se déplacer relativement l'une par rapport à l'autre que dans un seul sens de rotation.

Dans le sens de vissage, les flancs à pente montante de la partie d'accrochage glissent contre les flancs à pente montante de la partie d'expansion pour permettre le mouvement relatif en rotation, et par conséquent en translation selon l'axe longitudinal de l'implant, de la partie d'accrochage par rapport à la partie d'expansion.

En cas de dévissage, c'est à dire dans le sens de rotation inverse au sens de vissage, les flancs à pente descendante de la partie d'accrochage viennent se positionner en butée contre les flancs à pente descendante de la partie d'expansion, bloquant par là même le dévissage.

Selon une caractéristique avantageuse de l'invention, la partie d'expansion comportent des gorges longitudinales aptes à permettre le blocage de l'implant dans le sens du vissage lorsque la mise en compression dudit implant dans le tissu osseux est achevée.

L'implant est ainsi immobilisé en rotation, dans le sens du vissage par les gorges longitudinales et dans le sens du dévissage par les moyens anti-retour.

Avantageusement, pour faciliter l'usinage du dispositif de fixation, les moyens de vissage coopérants et les moyens anti-retour sont dissociés les uns dans les autres.

De préférence, l'implant endo-osseux est réalisé dans un matériau biocompatible approprié, chirurgicalement acceptable, tel que par exemple le titane ou tout autre matériau implantable. L'implant endo-osseux est réalisé à partir d'une ébauche, par des méthodes connues, telles que par exemple par moulage, frittage, ou usinage.

En variante, l'implant endo-osseux peut être réalisé en zircone, à partir d'oxyde de zirconium. Il s'agit de préférence de zircone de qualité dentaire ou chirurgicale. Les implants endo-osseux en zircone présente un taux de biocompatibilité largement supérieur au titane et induisent une consolidation et une ostéointégration très rapides, de l'ordre de 4 fois plus rapide que des implants endo-osseux en titane.

Les implants endo-osseux en zircone évitent des contacts électriquement conducteurs avec le nerf dentaire et permettent une meilleure apparence esthétique, n'étant pas visibles sous la paroi de la prothèse dentaire qui est souvent mince et translucide.

Les implants endo-osseux selon l'invention présentent ainsi une très grande solidité, jointe à une excellente ostéointégration et à un traumatisme minime à la pose.

L'invention sera maintenant plus précisément décrite dans le cadre de modes de réalisation préférés, qui n'en sont nullement limitatifs, représentés sur les figures 3a à 12 dans lesquelles :
la figure 3a représente une vue de face d'un implant endo-osseux selon un premier mode de réalisation de l'invention, dans un état initial, avant insertion dans un logement d'un tissu osseux,
la figure 3b représente une vue de face de l'implant endo-osseux selon le premier mode de réalisation de l'invention, dans un état expansé, après insertion dans un logement d'un tissu osseux,
la figure 3c représente une vue en perspective de la partie d'accrochage de l'implant endo-osseux selon le premier mode de réalisation de l'invention
la figure 3d représente une vue en perspective de l'implant endo-osseux sans la partie d'accrochage illustrant une partie d'expansion de l'implant endo-osseux, selon le premier mode de réalisation de l'invention, la figure 3e illustre un agrandissement de l'implant endo-osseux de la figure 3d au niveau de la partie d'expansion,
Les figures 4a à 4c représentent respectivement une vue de face de l'implant, une coupe longitudinale et une coupe transversale du dispositif de fixation, selon une variante de réalisation du premier mode de réalisation de l'invention, dans un état initial, avant insertion dans un logement d'un tissu osseux,
Les figures 5a à 5c représentent respectivement une vue de face de l'implant, une coupe longitudinale et une coupe transversale du dispositif de fixation, selon une variante de réalisation du premier mode de réalisation de l'invention, dans un état expansé, après insertion dans un logement d'un tissu osseux,
Les figures 6a et 6b représentent une vue en perspective de l'implant sans sa partie d'accrochage et une coupe transversale au niveau de la partie d'expansion selon une variante de réalisation du premier mode de réalisation de l'invention,
Les figures 7a à 7c représentent deux vue en perspective et une coupe transversale de la partie d'accrochage associée à la partie d'expansion de l'implant des figues 6a et 6b,
Les figures 8a et 8b illustrent l'implantation de l'implant selon une variante de réalisation du premier mode de réalisation de l'invention dans un logement d'un tissu osseux, dans un état initial et dans un état expansé,
Les figures 9a et 9b illustrent une prothèse de la main ou du pied réalisée à partir d'un implant selon l'invention,
La figure 10 illustre une prothèse du genou réalisée à partir d'implants selon l'invention,
La figure 11 illustre une prothèse de hanche réalisée à partir d'un implant selon l'invention,
La figure 12 illustre une prothèse d'épaule réalisée à partir d'un implant selon l'invention.

Les différents modes de réalisation d'implants endo-osseux selon l'invention sont décrits ci-après, à titre non limitatif de l'invention, dans le cas d'une application dentaire.

Les implants endo-osseux dentaires sont destinés à être implantés, par un praticien, dans un tissu osseux de la mâchoire d'un patient, tel que par exemple un os de la mandibulaire ou maxillaire.

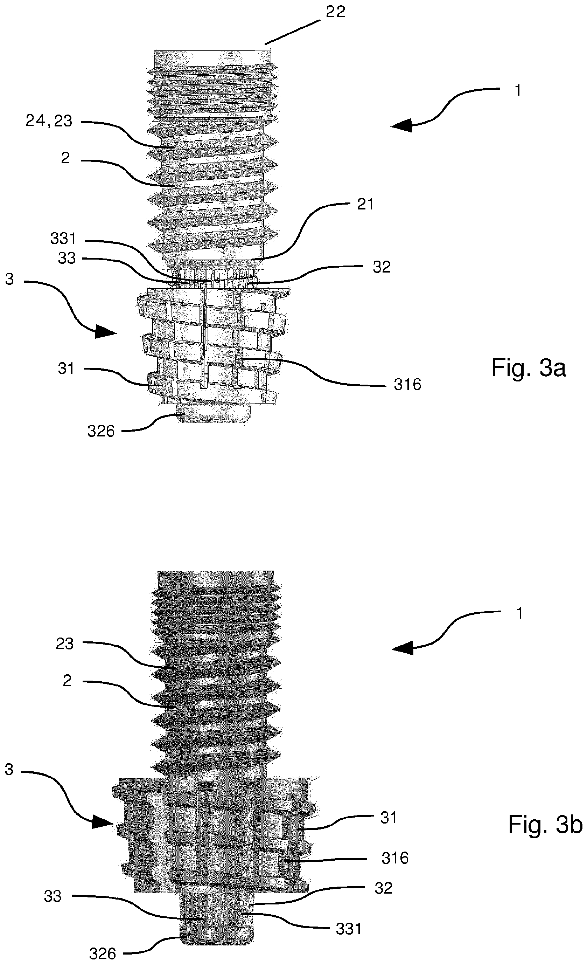

Les implants endo-osseux, appelés par contration implants 1, représentés aux figures 3a à 3d et illustrant un mode de réalisation comportent en commun une partie formant corps 2 formée d'une partie cylindrique, sensiblement de révolution autour d'un axe longitudinal 11, et de section transversale préférentiellement circulaire.

Le corps 2 comporte une première extrémité, dite extrémité endo-osseuse 21 et une seconde extrémité opposée, dite extrémité dite exo-osseuse 22.

De façon courante, l'implant est inséré dans un logement de réception 61 préalablement crée dans un tissu osseux 6 de la mâchoire de manière à laisser apparaître à la surface de la gencive l'extrémité exo-osseuse 22 de l'implant 1 permettant la mise en place d'un dispositif prothétique (non représenté sur les figures), tel qu'une prothèse dentaire, un moignon.

L'extrémité exo-osseuse 22 du corps forme une tête de raccordement destinée à recevoir le dispositif prothétique et comporte des premiers moyens de connexion 25 aptes à coopérer avec un outil de vissage pour l'insertion de l'implant 1 dans le logement 61.

Les moyens de connexion 25 sont par exemple un canal intérieur fileté et taraudé dans le corps débouchant au niveau de l'extrémité exo-osseuse 22.

Dans un autre exemple, les moyens de connexion sont une cavité interne polygonale, typiquement hexagonale, pour recevoir un outil de vissage tel qu'un tournevis ou une clé à six pans.

Le corps 2 comporte, au niveau d'une surface externe 24, des moyens d'ancrage 23.

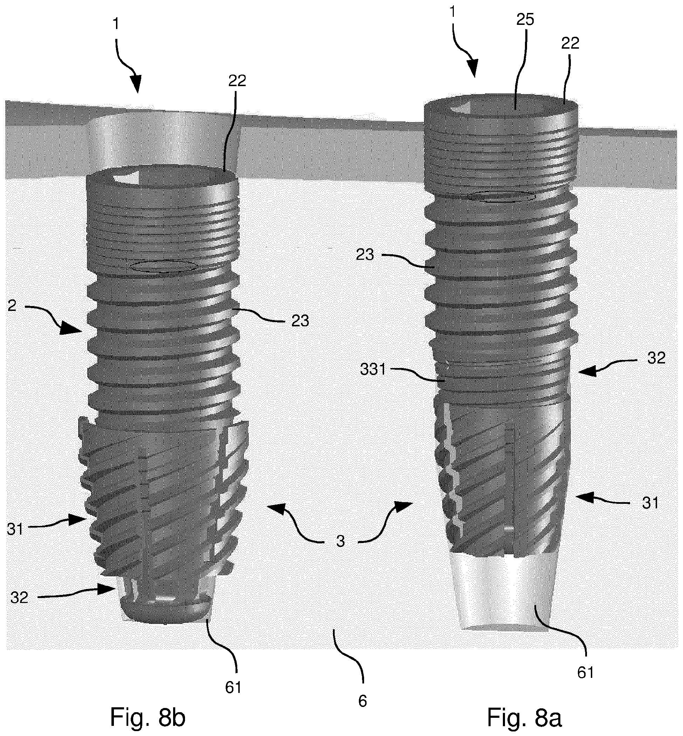

De préférence, les moyens d'ancrage 23 sont du type par vissage et comportent un filetage de pas constant et déterminé, par exemple compris entre 0,25 et 2mm, sur la surface externe 24 (le filetage est clairement représenté sur les figures 3a, 3b, 8a et 8b).

Les moyens d'ancrage 23 sont repartis sur toute ou partie d'une longueur du corps, préférentiellement sensiblement sur toute la longueur, comme illustré sur les figures.

La présence du filetage 23 sur l'implant 1 génère un mouvement hélicoïdal à l'ensemble de l'implant. Ce mouvement hélicoïdal permet de verrouiller l'implant dans une position en créant une compression dans le tissu osseux de la mâchoire.

La présence du filetage 23 sur l'implant permet également avantageusement d'augmenter considérablement la surface ostéointégrable de l'implant.

Le corps 2 se poursuit, au niveau de l'extrémité endo-osseuse 21, par un dispositif de fixation 3, de forme globalement tronconique s'amincissant en s'éloignant du corps 2.

Le dispositif de fixation 3 comporte :
- une première partie, dite partie d'accrochage 31 dans le tissu osseux,
- une deuxième partie, dite partie d'expansion 32,
- des moyens 33, 33a, 33b de liaison mécanique coopérants entre la partie d'accrochage 31 et la partie d'expansion 32.

Les moyens 33, 33a, 33b de liaison mécanique sont disposés d'une part au niveau de la partie d'accrochage 31 et d'autre part au niveau de la partie d'expansion 32.

Les moyens 33 de liaison mécanique permettent une mobilité relative des deux parties 31, 32 avec au moins un degré de liberté. En l'occurrence, pour tous les modes décrits ici, les deux parties 31, 32 sont mobiles l'une par rapport à l'autre en translation suivant l'axe longitudinal 11.

Le mouvement de translation génère un déplacement relatif desdites deux parties provoquant un élargissement de la partie d'accrochage 31 radialement par rapport à l'axe longitudinal 11. L'élargissement radial entraîne l'accrochage de la partie d'accrochage 31 sur des parois latérales du logement dans le tissu osseux. Le dispositif de fixation 3 peut prendre différentes formes. A titre d'exemples non restrictif de l'invention, des formes vont être à présent décrites et illustrées sur les figures 3a à 3e et 4a à 8b. La partie d'expansion 32 est liée à l'extrémité endo-osseuse 21 du corps 2 de l'implant 1 et se présente sous une forme tronconique se rétrécissant à partir de ladite extrémité endo-osseuse.

La partie d'accrochage 31 est agencée circonférentiellement autour de la partie d'expansion 32 et présente une surface interne 318 coopérant avec une surface externe 327 de la partie d'expansion 32.

Pour solidariser la partie d'accrochage 31 à la partie d'expansion 32, ladite partie d'expansion présente en outre un épaulement 326 à son extrémité la plus petite.

La partie d'accrochage 31 est réalisée dans un matériau biocompatible qui présente des propriétés élastiques permettant, lorsque la partie d'accrochage est sollicité élastiquement, de s'élargir radialement entre sa position initiale, avant introduction dans le logement et sa position déployée, après introduction dans le logement.

Dans ce mode de réalisation, l'implant est préférentiellement réalisé en titane ou en zircone.

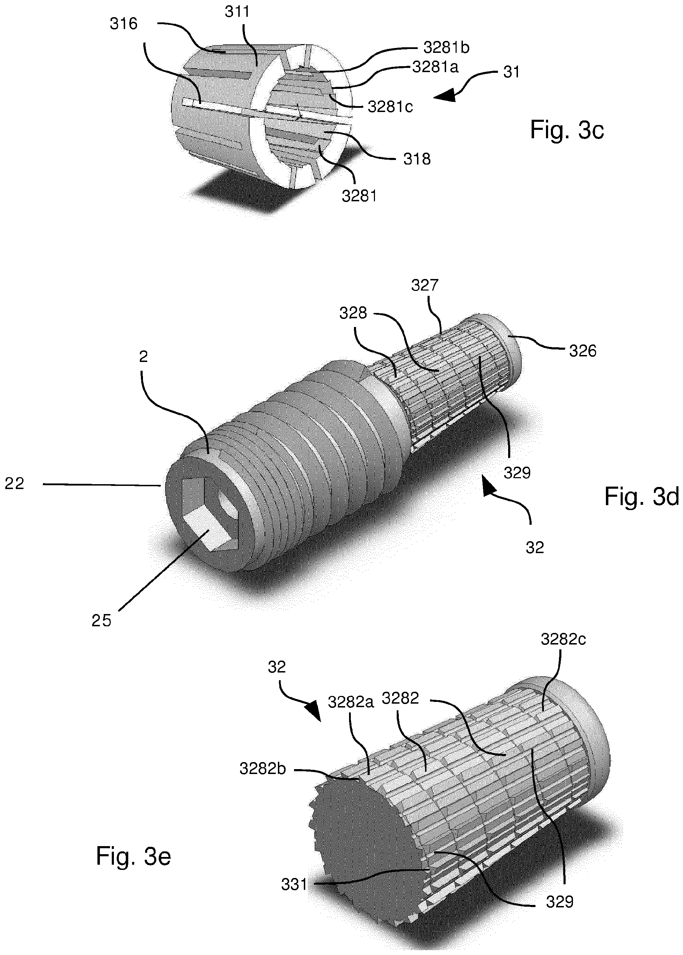

Elle comporte, dans illustré dans l'exemple de la figure 3c, des fentes longitudinales 316 débouchant au niveau de l'une ou de l'autre de deux extrémités longitudinales de la partie d'accrochage 31, aptes à permettre, en plus des propriétés élastiques intrinsèques au matériau de la partie d'accrochage 31, un élargissement encore plus important de ladite partie d'accrochage.

Bien que les fentes soient illustrées sur la figure 3c et décrites au nombre de douze, le nombre de ces fentes n'est pas limité à celui décrit et illustré. Ainsi, il est possible, sans se départir du cadre de l'invention, de réaliser une partie d'accrochage 31 avec deux, trois fentes voir plus.

Elle comporte, dans un autre exemple non représenté, quatre éléments reliés deux à deux par deux branches légèrement courbées, aptes à permettre, en plus des propriétés élastiques intrinsèques au matériau de la partie d'accrochage 31, un élargissement encore plus important de ladite partie d'accrochage.

Pour améliorer l'accroche de l'implant dans le tissu osseux, la partie d'accrochage 31 comporte des moyens d'immobilisation en rotation et translation sur tout ou partie d'une surface externe 311 de la partie d'accrochage 31. Afin de ne pas surcharger les figures, les moyens d' d'immobilisation en rotation et translation n'ont pas été représentés.

Dans un exemple de réalisation des moyens d'immobilisation, lesdits moyens sont une pluralité de lames ou saillies, d'orientation aléatoire, de section transversale préférentiellement triangulaire, et présentant une arête tranchante.

Selon une caractéristique de ce mode de réalisation, les moyens de liaison coopérants 33 permettant de maintenir la partie d'expansion 32 dans la partie d'accrochage 31 consistent en une liaison hélicoïdale, entre la surface externe 327 de la partie d'expansion 32 et la surface interne 318 de la partie d'accrochage 31.

Par exemple, la liaison hélicoïdale 33 est formée d'une part, au niveau de la surface externe 327 de la partie d'expansion 32, d'un filetage conique 331, de préférence de pas constant préférentiellement identique au pas du filetage 23 sur la surface externe 24 du corps 2, et d'autre part, au niveau de la surface interne 318 de la partie d'accrochage 31, d'un taraudage (non représenté sur les figures) de pas complémentaire.

Dans un mode de réalisation particulier, la surface externe 327 comporte un double filetage conique décalé de 180°.

La liaison hélicoïdale 33 permet d'entraîner la partie d'accrochage 31 en rotation dans un sens dit de vissage autour de la partie d'expansion 32 engendrant par là même sa translation le long de l'axe longitudinal 11, en direction du corps 2.

Préférentiellement, le dispositif de fixation 3 comporte en outre, sur la surface externe 327 de la partie d'expansion 32 et la surface interne 318 de la partie d'accrochage 31, des moyens anti-retour 328 coopérants, tels qu'illustrés sur les figures 3c, 3d et 3e, aptes à empêcher la rotation entre la partie d'accrochage et la partie d'expansion en sens inverse à celui imparti par la liaison hélicoïdale et donc à bloquer l'implant en rotation. Les moyens anti-retour 328 empêchent la rotation entre les deux parties en sens inverse du sens de vissage. Ainsi, l'implant ne peut pas se dévisser inopportunément.

Dans un exemple de réalisation des moyens anti-retour 328, lesdits moyens sont formés d'une part, au niveau de la surface externe 327 de la partie d'expansion 32, par une première denture périphérique extérieure 3282 et d'autre part, au niveau de la surface interne 318 de la partie d'accrochage 31, par une deuxième denture périphérique extérieure 3281. Lesdites deux dentures périphériques extérieures 3281, 3282 sont destinées à coopérer ensemble, que l'implant soit dans un état initial, c'est-à-dire non expansé, ou en cours d'expansion, ou dans un état expansé, c'est-à-dire verrouillé dans une position créant une compression dans le tissu osseux.

Dans une forme de réalisation préférentielle de l'invention, la denture périphérique extérieure 3281 de la partie d'accrochage 31, respectivement 3282 et de la partie d'expansion 32, présente un profil transversal en dents de scie à flancs asymétriques, avec des flancs à pente montante 3281 a, respectivement 3282a, et des flancs à pente descendante 3281 b, respectivement 3282b. Chaque flanc à pente montante est relié à un flanc à pente descendante par une arête sensiblement tranchante 3281 c, respectivement 3282c. Les dents des deux dentures périphériques extérieures 3281, 3282 sont orientées de manière à ce que dans le sens de vissage, la partie d'accrochage 31 est entraînée en rotation autour de la partie d'expansion 32 engendrant par là même sa translation selon l'axe longitudinal 11 de l'implant.

Les dentures périphériques extérieures 3281, 3282 sont donc disposées l'une par rapport à l'autre de sorte à ne pouvoir se déplacer relativement l'une par rapport à l'autre que dans un seul sens de rotation.

Lors du vissage, dans le sens de vissage, les flancs à pente montante 3281 a de la partie d'accrochage 31 glissent contre les flancs à pente montante 3282a de la partie d'expansion 32 pour permettre le mouvement relatif en rotation, et par conséquent en translation selon l'axe longitudinal 11 de l'implant, de la partie d'accrochage 31 par rapport à la partie d'expansion 32.

En cas d'hypothétique dévissage, c'est à dire dans le sens de rotation inverse au sens de vissage, les flancs à pente descendante 3281 b de la partie d'accrochage 31 viennent se positionner en butée contre les flancs à pente descendante 3282b de la partie d'expansion 32, bloquant, par là même, le dévissage.

Dans un exemple de réalisation des dents à la surface externe 327 de la partie d'expansion 32, les flancs à pente montante 3282a présentent un angle α, par rapport à une tangente à ladite surface externe, compris entre 0 et 45°. Les flancs à pente descendante 3282b présentent un angle β par rapport à une tangente à ladite surface externe, compris entre 55 et 90°.

Dans un exemple de réalisation des dents à la surface interne 318 de la partie d'accrochage 31, les flancs à pente montante 3281 a présentent un angle γ, par rapport à une tangente à ladite surface interne, compris entre 0 et 45°. Les flancs à pente descendante 3281 b présentent un angle δ, par rapport à une tangente à ladite surface interne, compris entre 55 et 90°.

Les flancs à pente descendante 3281 b, 3282b présentent chacun une hauteur telle que, lorsque l'implant est dans un état expansé maximal possible, les flancs à pente descendante 3281 b de la partie d'accrochage 31 viennent se positionner en butée contre les flancs à pente descendante 3282b de la partie d'expansion 32, bloquant par là même le dévissage. Cette hauteur, est dans un exemple non limitatif, comprise entre 0,3 et 5mm.

Le dispositif de fixation 3 comporte en outre, sur la surface externe 327 de la partie d'expansion 32, des gorges longitudinales 329 aptes à limiter le serrage de l'implant et ainsi à verrouiller la mise en pression sur la partie d'accrochage mobile 31.

Dans un exemple préféré de réalisation, les gorges longitudinales 329 sont réalisées chacune au niveau d'un flanc à pente montante 3282a d'une dent. Les gorges longitudinales 329 permettent ainsi de renforcer le blocage de l'implant dans le sens du vissage, lorsque la mise en pression est achevée.

Dans l'exemple de la figure 3e, les gorges longitudinales 329 sont sensiblement à mi-hauteur du flanc à pente montante 3282a.

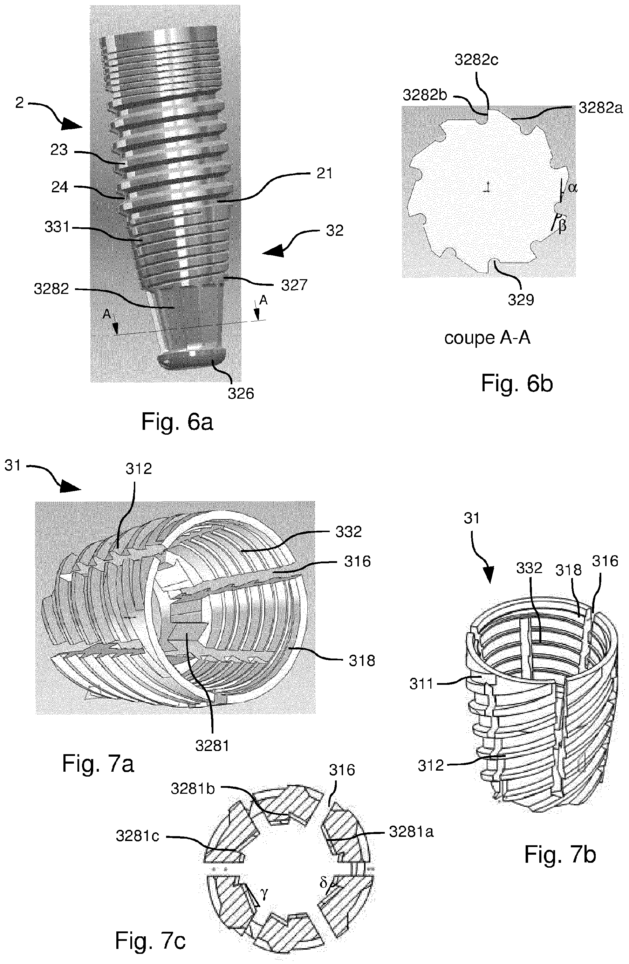

Dans l'exemple de la figure 6b, les gorges longitudinales 329 sont sensiblement à la base du flanc à pente montante 3282a.

L'implant est ainsi immobilisé autant en translation, par les moyens d'immobilisation, qu'en rotation, dans le sens du vissage par les gorges longitudinales 329 et dans le sens du dévissage par les moyens anti-retour 328.

Dans le mode de réalisation illustré sur les figures 3a à 3e, les moyens de liaison coopérants 33 sont imbriqués dans les moyens anti-retour 328.

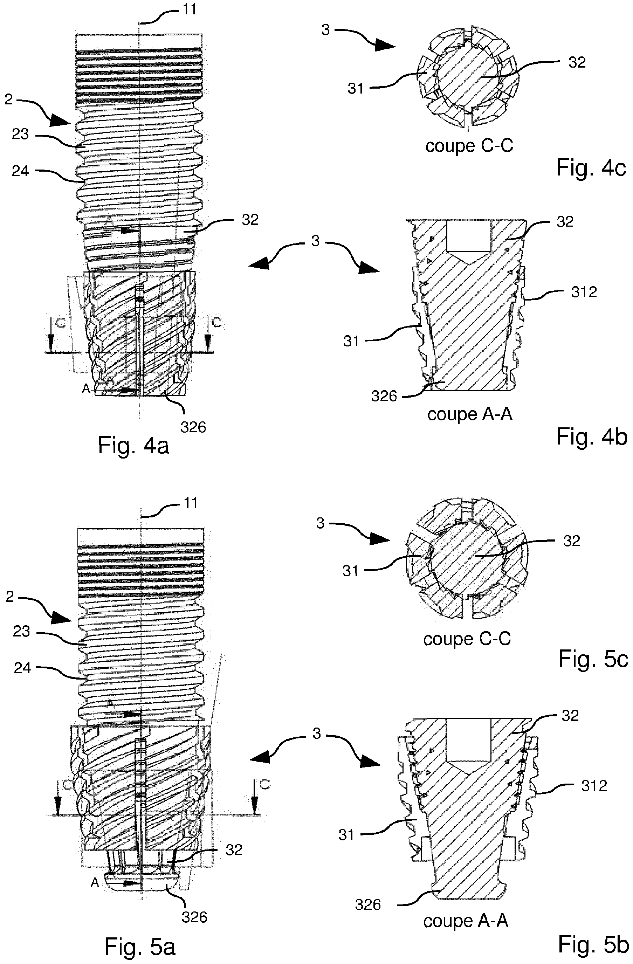

Dans une variante de réalisation de ce mode de réalisation du dispositif de fixation, illustrée par les figures 4a à 8b, les moyens de liaison coopérants 33 ne sont plus imbriqués dans les moyens anti-retour 328 mais en sont dissociés pour faciliter l'usinage de l'implant.

La partie d'expansion 32 se présente toujours sous une forme tronconique se rétrécissant à partir de ladite extrémité endo-osseuse.

La partie d'expansion 32 comporte, comme illustrée sur les figures 4a à 5c, d'une part, du coté de l'extrémité endo-osseuse 32, le filetage conique 331 de la liaison hélicoïdale 33, et d'autre part, du coté de l'épaulement 326, la denture périphérique extérieure 3282 à profil en dents de scie des moyens anti-retour 328.

Avantageusement, pour permettre un vissage accéléré de l'implant dans le logement, le filetage conique 331 est un filetage à double filets de type connu en soi.

La partie d'accrochage 31 comporte, comme illustrée sur les figures 6a à 7c, d'une part, à une extrémité située du coté de l'extrémité endo-osseuse lorsque la partie d'accrochage est en position sur l'implant, le taraudage 332 de la liaison hélicoïdale 33, et d'autre part, à une extrémité située du coté de l'épaulement 326 lorsque la partie d'accrochage est en position sur l'implant, la denture périphérique extérieure 3281 à profil en dents de scie des moyens anti-retour 328.

Lorsque la partie d'accrochage 31 est en position sur la partie d'expansion 32 de l'implant à l'état initial :
- une portion du taraudage 332 de la partie d'accrochage 31 est amorcée dans une portion du filetage 331 de la partie d'expansion 32,
- la denture périphérique extérieure 3281 de la partie d'accrochage 31 est en vis-à-vis de la denture périphérique extérieure 3282 de la partie d'expansion 32, flanc à pente montante 3281 a contre flanc à pente montante 3282a.

De préférence, la partie d'accrochage 31 de l'implant à l'état initial est amorcée au niveau du vissage sur sensiblement un tour.

Dans un exemple de réalisation des moyens d'immobilisation, illustrés entre autre sur les figures 7a, 7b, 8a et 8b, lesdits moyens sont un filetage 312 sur la surface externe 311 de la partie d'accrochage 31, De préférence, le filetage 312 présente un pas sensiblement identique au pas du filetage 23 sur la surface externe 24 du corps 2.

De préférence, et contrairement au filetage 312 représenté sur les figures, le filetage est un filetage contrarotatif par rapport au filetage 23 sur la surface externe 24 du corps 2 de l'implant, pour améliorer l'effet d'ancrage.

Mode de fonctionnement de ce mode de réalisation de l'implant et de sa variante de réalisation

L'implant 1 (figures 3a et 8a) est introduit dans le logement 61 du tissu osseux 6 par vissage des moyens d'ancrage 23 dans les parois latérales 62 dudit logement 61 avec un outil de vissage adapté. L'implant est immobilisé en translation.

Lorsque le moyen d'élargissement 322 arrive en butée contre la paroi inférieure 63 du logement 61, le praticien continue le vissage. Grace aux moyens de liaison 33 (liaison hélicoïdale), la partie d'accrochage 31 observe, via une rotation dans un sens de vissage, un mouvement en translation, par rapport à la partie d'expansion 32, selon l'axe longitudinal 11, en direction du corps 2 de l'implant 1. Ce mouvement relatif en translation selon l'axe longitudinal 11 provoque un élargissement des fentes et entraîne par conséquent un élargissement de la partie d'accrochage (figures 3b et 8b) jusqu'à son ancrage dans les parois latérales 62 du logement 61. Le dévissage est impossible grâce aux moyens anti-retour 328. L'implant est immobilisé en rotation.

Le vissage de l'implant 1 dans le logement 61 du tissu osseux 6 génère avantageusement le mouvement relatif de translation entre les deux parties.

L'implant est ainsi immobilisé autant en translation qu'en rotation.

Ainsi, dans ce mode de réalisation de l'implant, pour un diamètre initial de corps de 3.5 mm, il est possible d'obtenir avec les propriétés élastiques de la partie d'accrochage en zircone, un implant après expansion avec un diamètre, au niveau de ladite partie d'accrochage, de 5 mm.

Les différents modes de réalisation de l'invention proposés permettent avantageusement d'améliorer l'étanchéité et l'ancrage de l'implant endo-osseux. Il n'est plus nécessaire d'attendre six mois pour laisser l'os s'accrocher à l'implant et un dispositif prothétique provisoire peut être installé directement sur l'implant quelques heures après son implantation dans le tissu osseux.

De plus, les différents modes de réalisation proposés sont d'une réalisation aisée, par exemple par usinage, avec des tolérances de fabrication souhaitées les plus larges possibles sans affecter la qualité de l'ancrage global de l'implant dans le tissu osseux. Leur fabrication est donc à moindre coût.

Un autre avantage de ces différents modes de réalisation de l'implant réside dans le fait qu'il présente une durée de vie allongée dans le corps du patient.

En effet, avec les implants actuels, il s'avère nécessaire de les changer au minimum tous les deux ans car la structure osseuse du patient s'est modifiée et les implants ne sont plus suffisamment ancrés dans ladite structure osseuse et peuvent donc être susceptibles d'être retirés accidentellement. Avec les différents modes de réalisation de l'implant, il suffit de retirer le dispositif prothétique, de visser à nouveau l'implant de sorte à élargir la partie d'accrochage 31 jusqu'à créer une nouvelle compression dans le tissu osseux puis de reposer le dispositif prothétique.

Les différents modes de réalisation d'implants endo-osseux selon l'invention ont été décrits ci-avant, à titre non limitatif de l'invention, dans le cas d'un implant dentaire.

L'implant endo-osseux suivant l'invention est également adaptable à d'autres applications, à partir de modifications à la portée de l'homme du métier.

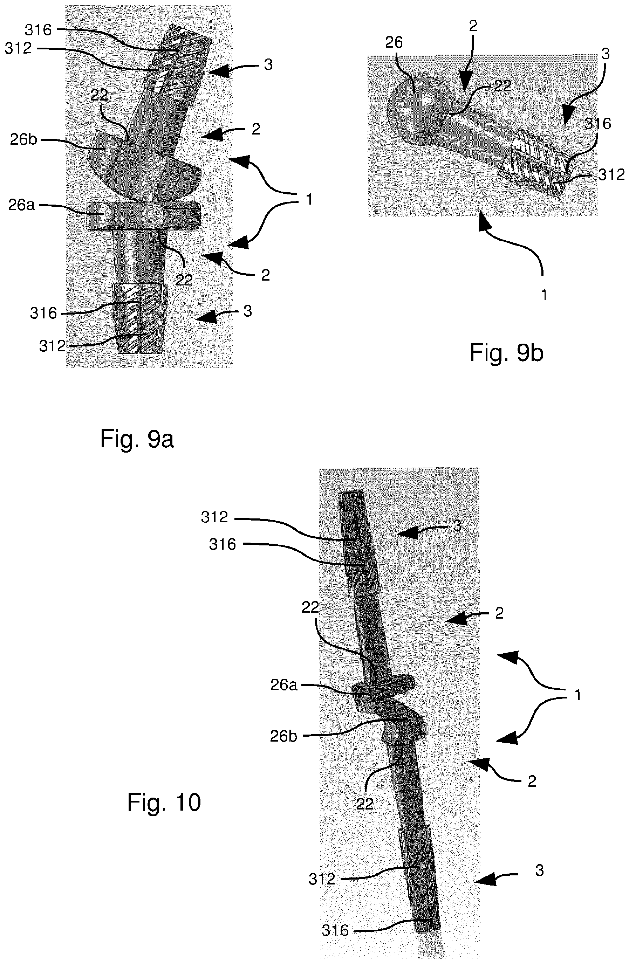

Dans un exemple d'application, illustré par les figures 9a et 9b, l'implant endo-osseux 1 est destiné à l'installation d'une prothèse articulaire au niveau d'une main ou d'un orteil d'un patient. La prothèse articulaire est une articulation artificielle qui va remplacer l'articulation abîmée due à une usure du cartilage, par exemple par de l'arthrose. Les articulations ciblées sont par exemple :
- pour la main, les articulations interphalangiennes distale ou proximale, les articulations métacarpophalangiennes,
- pour le pied, les articulations interphalangiennes distale ou proximale, les articulations métatarsophalangiennes.

La prothèse comporte, pour les articulations interphalangiennes distale ou proximale de la main ou du pied, deux implants endo-osseux dits phalangiens reliés par une liaison pivot simple, de type connu en soi, comme illustré sur la figure 9a.

Chaque implant phalangien comporte le corps 2 et le dispositif de fixation 3.

Dans un exemple de réalisation, les corps 2 des implants phalangiens comportent les moyens d'ancrage.

Dans un autre exemple de réalisation, tel qu'illustré sur les figures 9a et 9b, au contraire des implants dentaires, les corps 2 des implants phalangiens ne comportent pas de moyens d'ancrage.

Les dispositifs de fixation 3 comportent les parties d'accrochage et d'expansion ainsi que les moyens de liaison coopérant et les moyens anti-retour. Seule la partie d'expansion 31 avec ses fentes longitudinales 316 et les moyens d'immobilisation en rotation et translation 312 sont visibles sur la figure 9a.

Le corps 2 d'un des deux implants phalangiens 1 comporte à son extrémité exo-osseuse 22 un plateau sensiblement plat 26a et le corps du second implant phalangien 1 comporte à son extrémité exo-osseuse 22 un plateau sensiblement semi-sphérique 26b pour permettre le glissement entre les deux implants.

Chaque implant comporte un évidement longitudinal (non représenté sur la figure 9a), préférentiellement centré sur l'axe longitudinal de l'implant, débouchant au niveau de l'extrémité exo-osseuse, et permettant le passage d'un outil de vissage dudit implant.

Les implants phalangiens sont ancrés aux tissus osseux par leurs dispositifs de fixation 3 respectifs.

Dans un cas particulier d'application illustré figure 9b, la prothèse comporte, pour les articulations métacarpophalangiennes ou métatarsophalangiennes, un implant endo-osseux à liaison rotule, de type connu en soi.

L'implant comporte le corps 2 et le dispositif de fixation 3.

Comme pour les implants phalangiens :
- le dispositif de fixation 3 comporte les parties d'accrochage et d'expansion ainsi que les moyens de liaison coopérant et les moyens anti-retour. Seule la partie d'expansion 31 avec ses fentes longitudinales 316 et les moyens d'immobilisation en rotation et translation 312 sont visibles sur la figure 9b,
- l'implant comporte un évidement longitudinal (non représenté sur la figure 9b), préférentiellement centré sur l'axe longitudinal de l'implant, débouchant dans la tête sphérique, et permettant le passage d'un outil de vissage dudit implant.

Suivant deux exemples distincts de réalisation, le corps 2 comporte ou ne comporte pas de moyens d'ancrage,

Le corps 2 de l'implant 1 comporte à son extrémité exo-osseuse 22 une tête sphérique 26 pour permettre une liaison rotulienne avec une cupule creuse, de forme générale hémisphérique apte à être fixée dans le fond d'une cavité du métacarpe.

L'implant est ancré au tissu osseux par le dispositif de fixation 3.

### Mode de fonctionnement des implants articulaires de la main ou du pied dans l'exemple où le corps 2 ne comporte pas de moyens d'ancrage

Chaque implant 1 est introduit et positionné dans le logement, préalablement réalisé, du tissu osseux de la phalange, sans vissage jusqu'à la paroi inférieure. Le praticien introduit son outil de vissage dans l'évidement longitudinal et visse l'implant. Grace aux moyens de liaison coopérants, la partie d'accrochage 31 observe, via une rotation dans un sens de vissage, un mouvement en translation, par rapport à la partie d'expansion, selon l'axe longitudinal 11, en direction du corps 2 de l'implant 1. Ce mouvement relatif en translation selon l'axe longitudinal de l'implant provoque un élargissement des fentes et entraîne par conséquent un élargissement de la partie d'accrochage (figures 3b et 8b) jusqu'à son ancrage dans les parois latérales 62 du logement 61. Le dévissage est impossible grâce aux moyens anti-retour 328. L'implant est immobilisé en rotation et en translation dans la phalange.

Dans un autre exemple d'application, illustré par la figure 10, l'implant endo-osseux 1 est destiné à l'installation d'une prothèse de genou d'un patient.

La prothèse comporte un implant endo-osseux 1 dit implant fémoral et un implant endo-osseux 1 dit implant tibial reliés entre eux par une liaison pivot simple, de type connu en soi, comme illustré sur la figure 10.

Chaque implant tibial ou fémoral 1 comporte le corps 2 et le dispositif de fixation 3.

Suivant deux exemples distincts de réalisation, le corps 2 comporte ou ne comporte pas de moyens d'ancrage.

Les dispositifs de fixation 3 comportent les parties d'accrochage et d'expansion ainsi que les moyens de liaison coopérant et les moyens anti-retour. Seule la partie d'expansion 31 avec ses fentes longitudinales 316 et les moyens d'immobilisation en rotation et translation 312 sont visibles sur la figure 10.

Le corps 2 de l'implant tibial 1 comporte à son extrémité exo-osseuse 22 un plateau sensiblement plat 26a et le corps 2 de l'implant fémoral 1 comporte à son extrémité exo-osseuse 22 un plateau sensiblement semi-sphérique 26b pour permettre le glissement entre les deux implants.

Chaque implant comporte un évidement longitudinal (non représenté sur la figure 10), préférentiellement centré sur l'axe longitudinal de l'implant, débouchant au niveau de l'extrémité exo-osseuse, et permettant le passage d'un outil de vissage dudit implant.

Les implants sont ancrés aux tissus osseux par leurs dispositifs de fixation 3 respectifs.

### Mode de fonctionnement des implants du genou dans l'exemple où le corps 2 ne comporte pas de moyens d'ancrage

L'implant fémoral 1, respectivement tibial, est introduit et positionné dans le logement, préalablement réalisé, du tissu osseux du fémur, respectivement du tibia, sans vissage jusqu'à la paroi inférieure. Le praticien introduit son outil de vissage dans l'évidement longitudinal et visse. Grace aux moyens de liaison coopérants, la partie d'accrochage 31 observe, via une rotation dans un sens de vissage, un mouvement en translation, par rapport à la partie d'expansion, selon l'axe longitudinal 11, en direction du corps 2 de l'implant 1. Ce mouvement relatif en translation selon l'axe longitudinal de l'implant provoque un élargissement des fentes et entraîne par conséquent un élargissement de la partie d'accrochage (figures 3b et 8b) jusqu'à son ancrage dans les parois latérales 62 du logement 61. Le dévissage est impossible grâce aux moyens anti-retour 328. L'implant est immobilisé en rotation et translation dans le fémur, respectivement dans le tibia.

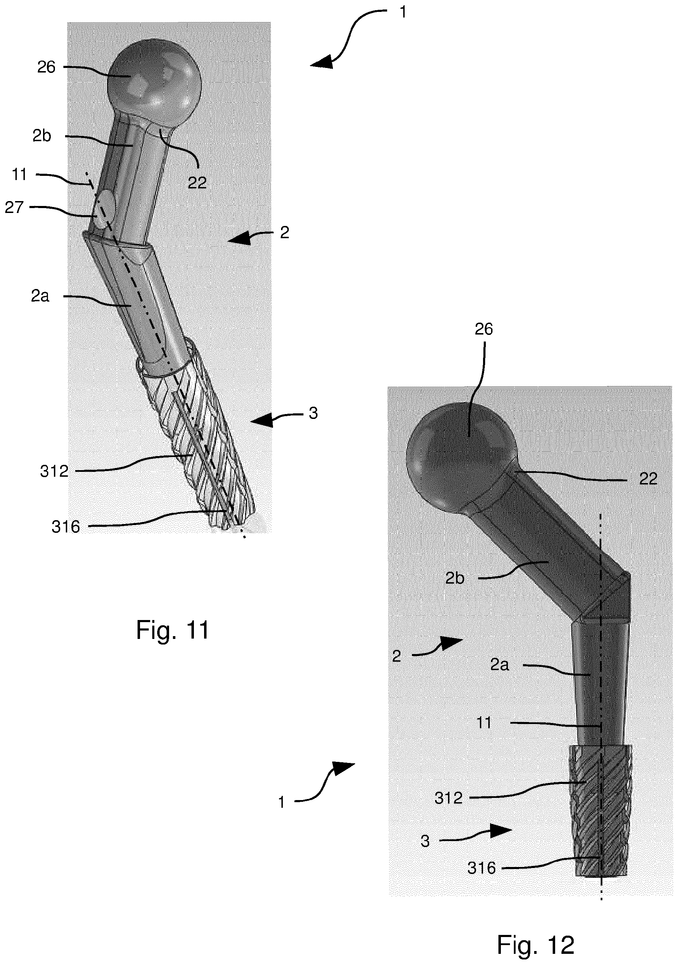

Dans un autre exemple d'application, illustré par les figures 11 et 12, l'implant endo-osseux 1 est destiné à l'installation d'une prothèse de hanche (figure 11) ou d'épaule (figure 12) d'un patient.

La prothèse comporte un implant endo-osseux, dit implant fémoral 1 pour la prothèse de hanche et implant huméral 1 pour la prothèse d'épaule, à liaison rotule, de type connu en soi.

L'implant fémoral, respectivement huméral, comporte le corps 2 et le dispositif de fixation 3. Comme pour les implants phalangiens, le dispositif de fixation 3 comporte les parties d'accrochage et d'expansion ainsi que les moyens de liaison coopérant et les moyens anti-retour. Seule la partie d'expansion 31 avec ses fentes longitudinales 316 et les moyens d'immobilisation en rotation et translation 312 sont visibles sur la figure 11.

Suivant deux exemples distincts de réalisation, le corps 2 comporte ou ne comporte pas de moyens d'ancrage,

Le dispositif de fixation 3 est destiné à s'étendre dans la diaphyse du fémur, respectivement de l'humérus. Le corps 2 comporte une partie diaphysaire 2a destinée à s'étendre dans la diaphyse du fémur, respectivement de l'humérus, dans le prolongement du dispositif de fixation 3, et une partie métaphysaire 2b destinée à s'étendre dans la métaphyse du fémur, respectivement de l'humérus, avec un angle prédéfini par rapport à l'axe longitudinal 11.

Le corps 2 de l'implant fémoral 1, respectivement huméral, comporte à son extrémité exo-osseuse 22 une tête sphérique 26 pour permettre une liaison rotulienne avec une cupule creuse, de forme générale hémisphérique apte à être fixée dans le fond d'une cavité de l'os iliaque, respectivement de l'omoplate.

L'implant fémoral, respectivement huméral, comporte un évidement longitudinal 27, préférentiellement centré sur l'axe longitudinal de l'implant, débouchant dans la partie métaphysaire 2b, et permettant le passage d'un outil de vissage dudit implant.

L'implant fémoral, respectivement huméral, est ancré au tissu osseux du fémur par le dispositif de fixation 3.

### Mode de fonctionnement de l'implant de la prothèse de la hanche, respectivement de l'épaule, dans l'exemple où le corps 2 ne comporte pas de moyens d'ancrage

L'implant fémoral 1, respectivement huméral, est introduit et positionné dans le logement, préalablement réalisé, du tissu osseux du fémur, respectivement de l'humérus, sans vissage jusqu'à la paroi inférieure. Le praticien introduit son outil de vissage dans l'évidement longitudinal et visse. Grace aux moyens de liaison coopérants, la partie d'accrochage 31 observe, via une rotation dans un sens de vissage, un mouvement en translation, par rapport à la partie d'expansion, selon l'axe longitudinal 11, en direction du corps 2 de l'implant 1. Ce mouvement relatif en translation selon l'axe longitudinal de l'implant provoque un élargissement des fentes et entraîne par conséquent un élargissement de la partie d'accrochage (figures 3b et 8b) jusqu'à son ancrage dans les parois latérales 62 du logement 61. Le dévissage est impossible grâce aux moyens anti-retour 328. L'implant est immobilisé en rotation et translation dans le fémur, respectivement dans l'humérus.

## Revendications

1. Implant endo-osseux (1), apte à être implanté dans un tissu osseux, comprenant un corps (2) présentant une forme allongée selon un axe longitudinal (11) et un dispositif de fixation (3) lié à une des extrémités dudit corps et comportant :
- une partie, dite d'accrochage (31) dans le tissu osseux,
- une partie, dite d'expansion (32), lesdites deux parties étant mobiles l'une par rapport l'autre,
- des moyens (33, 33a, 33b) de liaison mécanique coopérants, disposés d'une part au niveau de la partie d'accrochage (31) et d'autre part au niveau de la partie d'expansion (32), tels que la mobilité relative des deux parties comprend au moins un degré de liberté et tels qu'un déplacement relatif desdites deux parties provoque un élargissement de la partie d'accrochage (31), ledit élargissement entraînant l'accrochage de la partie d'accrochage dans le tissu osseux,
la partie d'expansion (32) étant fixée au corps (2) et la partie d'accrochage (31) étant agencée circonférentiellement autour de ladite partie d'expansion,
les moyens de liaison (33) étant des moyens de vissage coopérants permettant une rotation entre la partie d'accrochage (31) et la partie d'expansion dans un sens et **caractérisé en ce que** le dispositif de fixation (3) comporte des moyens anti-retour (328) coopérants aptes à empêcher une rotation entre la partie d'accrochage (31) et la partie d'expansion (32) en sens inverse à celui imparti par lesdits moyens de vissage.

2. Implant endo-osseux (1) selon la revendication 1 **caractérisé en ce que** le au moins un degré de liberté est une translation selon l'axe longitudinal (11).

3. Implant endo-osseux (1) selon l'une des revendications précédentes dans lequel la partie d'accrochage (31) est déformable élastiquement et comporte au moins deux fentes longitudinales (316).

4. Implant endo-osseux (1) selon l'une des revendications précédentes dans lequel la partie d'accrochage (31) comporte, au niveau d'une surface externe (311), des moyens d'immobilisation (312) en rotation et/ou en translation dudit implant endo-osseux dans le tissu osseux après l'implantation.

5. Implant endo-osseux (1) selon l'une des revendications précédentes dans lequel les moyens anti-retour (328) coopérants comportent d'une part, au niveau d'une surface externe (327) de la partie d'expansion (32), une première denture périphérique extérieure (3282) et d'autre part, au niveau d'une surface interne (318) de la partie d'accrochage (31), une deuxième denture périphérique extérieure (3281).

6. Implant endo-osseux (1) selon la revendication 5 dans lequel les dentures périphériques extérieures (3281, 3282) de la partie d'accrochage (31) et de la partie d'expansion (32), ont un profil transversal en dents de scie à flancs asymétriques.

7. Implant endo-osseux (1) selon l'une des revendications précédentes dans lequel la partie d'expansion (32) comportent des gorges longitudinales (329) aptes à permettre le blocage de l'implant dans le sens du vissage lorsque la mise en compression dudit implant dans le tissu osseux est achevée.

## Patentansprüche

1. Enossales Implantat (1), das zur Implantierung in ein Knochengewebe geeignet ist, umfassend einen Körper (2), der eine längliche Form entlang einer Längsachse (11) aufweist, und eine Fixierungsvorrichtung (3), die mit einem der Enden des Körpers verbunden ist und Folgendes aufweist:
- einen so genannten Anbringteil (31) im Knochengewebe,
- einen so genannten Expansionsteil (32), wobei die beiden Teile bezüglich einander beweglich sind,
- zusammenwirkende mechanische Verbindungsmittel (33, 33a, 33b), die einerseits auf der Höhe des Anbringteils (31) und andererseits auf der Höhe des Expansionsteils (32) angeordnet sind, so dass die relative Beweglichkeit der beiden Teile mindestens einen Freiheitsgrad umfasst und dass eine Relativverschiebung der beiden Teile eine Aufweitung des Anbringteils (31) hervorruft, wobei die Aufweitung zum Anbringen des Anbringteils im Knochengewebe führt,
wobei der Expansionsteil (32) am Körper (2) befestigt ist und der Anbringteil (31) umfangsmäßig um den Expansionsteil herum angeordnet ist, wobei die Verbindungsmittel (33) zusammenwirkende Schraubmittel sind, die eine Drehung zwischen dem Anbringteil (31) und dem Expansionsteil in einer Richtung gestatten, **dadurch gekennzeichnet, dass** die Fixierungsvorrichtung (3) zusammenwirkende Rückdrehsicherungsmittel (328) aufweist, die geeignet sind, eine Drehung zwischen dem Anbringteil (31) und dem Expansionsteil (32) in einer Richtung zu verhindern, die der durch die Schraubmittel verliehenen entgegengesetzt ist.

2. Enossales Implantat (1) nach Anspruch 1, **dadurch gekennzeichnet, dass** der mindestens eine Freiheitsgrad eine Translation entlang der Längsachse (11) ist.

3. Enossales Implantat (1) nach einem der vorhergehenden Ansprüche, wobei der Anbringteil (31) elastisch verformbar ist und mindestens zwei Längsschlitze (316) aufweist.

4. Enossales Implantat (1) nach einem der vorhergehenden Ansprüche, wobei der Anbringteil (31) auf der Höhe einer Außenfläche (311) Mittel (312) zur rotationsmäßigen und/oder translationsmäßigen Festlegung des enossalen Implantats im Knochengewebe nach der Implantation aufweist.

5. Enossales Implantat (1) nach einem der vorhergehenden Ansprüche, wobei die zusammenwirkenden Rückdrehsicherungsmittel (328) auf der Höhe einer Außenfläche (327) des Expansionsteils (32) einerseits eine erste äußere Umfangszahnung (3282) und auf der Höhe einer Innenfläche (318) des Anbringteils (31) andererseits eine zweite äußere Umfangszahnung (3281) aufweisen.

6. Enossales Implantat (1) nach Anspruch 5, wobei die äußeren Umfangszahnungen (3281, 3282) des Anbringteils (31) und des Expansionsteils (32) ein Querprofil aus Sägezähnen mit asymmetrischen Flanken haben.

7. Enossales Implantat (1) nach einem der vorhergehenden Ansprüche, wobei der Expansionsteil (32) Längsnuten (329) aufweist, die geeignet sind, die Blockierung des Implantats in der Schraubrichtung zu gestatten, wenn die Komprimierung des Implantats im Knochengewebe erreicht ist.

## Claims

1. Endosseous implant (1) capable of being implanted in bone tissue, comprising a body (2), elongate in shape along a longitudinal axis (11), and a fastening device (3) connected to one of the ends of said body and having:
- a gripping part (31) for gripping in the bone tissue,
- an expansion part (32), said two parts being movable in relation to each other,
- engaging mechanical connection means (33, 33a, 33b) arranged, on one end, at the gripping part (31) and, on the other end, at the expansion part (32), such that the relative mobility of the two parts comprises at least one degree of freedom, and such that a relative movement of said two parts causes a widening of the gripping part (31), said widening causing the gripping part to grip in the bone tissue,
the expansion part (32) being fastened to the body (2), and the gripping part (31) being arranged circumferentially around said expansion part,
the connection means (33) being engaging screwing means permitting a rotation between the gripping part (31) and the expansion part in one direction, and **characterized in that** the fastening device (3) has engaging non-return means (328) capable of preventing rotation between the gripping part (31) and the expansion part (32) in a direction opposite to that imparted by said screwing means.

2. Endosseous implant (1) according to Claim 1, **characterized in that** the at least one degree of freedom is a translation along the longitudinal axis (11).

3. Endosseous implant (1) according to either of the preceding claims, in which the gripping part (31) is elastically deformable and has at least two longitudinal slits (316).

4. Endosseous implant (1) according to one of the preceding claims, in which the gripping part (31) has, on an outer surface (311), immobilizing means (312) for immobilizing said endosseous implant in rotation and/or translation within the bone tissue after implantation.

5. Endosseous implant (1) according to one of the preceding claims, in which the engaging non-return means (328) have, on the one hand, on an outer surface (327) of the expansion part (32), a first outer peripheral toothing (3282), and, on the other hand, on an inner surface (318) of the gripping part (31), a second outer peripheral toothing (3281).

6. Endosseous implant (1) according to Claim 5, in which the outer peripheral toothings (3281, 3282) of the gripping part (31) and of the expansion part (32) have a saw-tooth transverse profile with asymmetrical flanks.

7. Endosseous implant (1) according to one of the preceding claims, in which the expansion part (32) has longitudinal grooves (329) capable of allowing the implant to be blocked in the screwing direction when the compression of said implant within the bone tissue is completed.
